# EUROPEAN PATENT APPLICATION

(11) **EP 3 794 952 A1**
(43) Date of publication of application: **24.03.2021**
(21) Application number: 19198414.5
(22) Date of filing: 19.09.2019
(51) Int. Cl.: A23K 10/18, A23K 20/174, A23K 50/30, A23K 50/60, A23K 50/70, A23K 50/75, A61K 35/742, A61K 31/355, A23L 33/135, A61P 1/14, C12R 1/10, C12R 1/125

(54) **FEED ADDITIVE COMPRISING BACILLUS STRAINS FOR INCREASING VITAMINE E ABSORPTION**

(71) Applicant: DSM IP Assets B.V., 6411 TE Heerlen (NL)
(72) Inventor: Perez Calvo, Estefania, 4303 Kaiseraugst (CH)
(74) Representative: Schwander, Kuno

(57) **Abstract**

It has been found that administering probiotic Bacillus strains to animals improves vitamin E absorption. Thus, probiotic Bacillus strains can be used when animals are stressed, e.g. during weaning. The present invention relates to a Bacillus strain as a feed additive, which is useful for improving vitamin E absorption in animals.

## Description

### REFERENCE TO A SEQUENCE LISTING

This application contains a Sequence Listing in computer readable form, which is incorporated herein by reference.

### Field of the Invention

The present invention generally relates to the field of meat producing animals. In particular, it relates to a food supplementation composition for meat producing animals containing one or more probiotic Bacillus strains. More particularly, the invention relates to a food supplementation composition containing the above-mentioned ingredients for the improvement of vitamine E absorption in the meat producing animal, more specifically a swine.

### Background of the invention

The pig experiences biological stress when being weaned from the sow. During this time, pigs are subjected to a number of stressors, such as an abrupt separation from the sow, relocation and handling stress, a radical change in diet, social hierarchy stress, mixing with piglets from other litters, a novel physical environment, increased exposure to pathogens and dietary or environmental antigens. This process can predispose the pig to intestinal and immune system dysfunctions and result in reduced pig health, growth, feed intake and increased mortality, particularly during the first week after weaning. These problems occurring after weaning are widely spread and cause severe economic losses to the pig producer. It is therefore important to ameliorate stress-induced problems in the pig.

### Summary of the invention

Pigs are naturally stressed during and after weaning and susceptible to stress-induced problems in health and performance. To avoid economic losses to the pig producer, it is imperative to minimize the impact of stress on performance in the pig. Nutrition plays an important role in in stress control in young pigs. Antioxidants, in particular vitamin E, are known to support immune function and help the pig to cope with stress and the linked suppression of the immune system. Therefore, a good absorption of Vit E from the diet is highly relevant for the health status and performance of the animals. In accordance with the present invention it has now been found that vitamin E absorption can be substantially increased by administering to the animals an effective amount of one or more probiotic Bacillus strains.

The present invention relates to the use of one or more probiotic Bacillus strains in the manufacture of a food composition for the improvement of vitamin E absorption in the meat producing animal, more specifically a swine, most specifically a weaned piglet, compared to untreated animals.

More particularly, the invention relates to the use of one or more probiotic Bacillus strains chosen from the group of Bacillus subtilis and Bacillus licheniformis in the manufacture of a food composition for the improvement of vitamin E absorption in the meat producing animal, more specifically a swine, most specifically a weaned piglet, compared to untreated animals.

According to the present invention the improvement of vitamin E absorption is characterized by an increase of vitamin E in the plasma by at least 20%, more preferably by at least 30%, more preferably by at least 40%, more preferably by at least 50% and most preferably by at least 60%, for example 63%.

According to the present invention the improvement of vitamin E absorption is characterized by an increase of vitamin E in the plasma to a value above 900 [ng/ml], most preferably above 1000 [ng/ml], for example 1051 [ng/ml].

In another aspect, the invention relates to a method for the improvement of vitamin E absorption in a meat producing animal, more specifically a swine, most specifically a weaned piglet, which comprises administering to an animal in need of such treatment one or more probiotic Bacillus strains.

In a more specific embodiment, the invention relates to a method for the improvement of vitamin E absorption in a meat producing animal, more specifically a swine, most specifically a weaned piglet, which comprises administering to an animal in need of such treatment one or more probiotic Bacillus strains chosen from the group of Bacillus subtilis and Bacillus licheniformis.

More specifically, the invention relates to a method for improving vitamin E absorption in tha plasma of a meat producing animal, more specifically a swine, most specifically a weaned piglet to a value above 900 [ng/ml], most preferably above 1000 [ng/ml], for example 1051 [ng/ml], which comprises administering to an animal in need of such treatment one or more probiotic Bacillus strains.

More specifically, the invention relates to a method for improving vitamin E absorption in the plasma of a meat producing animal, more specifically a swine, most specifically a weaned piglet to a value above 900 [ng/ml], most preferably above 1000 [ng/ml], for example 1051 [ng/ml], which comprises administering to an animal in need of such treatment one or more probiotic Bacillus strains chosen from the group of Bacillus subtilis and Bacillus licheniformis.

More specifically, the invention relates to a method for improving vitamin E absorption in tha plasma of a meat producing animal, more specifically a swine, most specifically a weaned piglet by at least 20%, more preferably by at least 30%, more preferably by at least 40%%, more preferably by at least 50% and most preferably by at least 60%, which comprises administering to an animal in need of such treatment one or more probiotic Bacillus strains.

More specifically, the invention relates to a method for improving vitamin E absorption in tha plasma of a meat producing animal, more specifically a swine, most specifically a weaned piglet by at least 20%, more preferably by at least 30%, more preferably by at least 40%%, more preferably by at least 50% and most preferably by at least 60%, which comprises administering to an animal in need of such treatment one or more probiotic Bacillus strains chosen from the group of Bacillus subtilis and Bacillus licheniformis.

A feed supplementation composition containing one or more probiotic Bacillus strains is therefore one preferred embodiment of the present invention.

In one preferred embodiment of the invention the one or more probiotic Bacillus strains is chosen from the group of Bacillus subtilis and Bacillus licheniformis.

According to the present invention the preferred animal is a monogastric animal, more specifically a meat producing animal, more specifically a swine, most specifically a weaned piglet.

According to the present invention one or more probiotic Bacillus strains are suitably administered together with the feed. Feed may be supplemented by admixing one or more of the probiotic Bacillus strains according to the present invention to regular feed or by first preparing a premix of a feed component and one or more probiotic Bacillus strains and subsequent mixing the premix with other feed components. The feed can be any conventional animal feed. The terms "feed" or "feed composition" as used herein comprise solid and liquid food as well as drinking fluids such as drinking water.

Generally, one or more probiotic Bacillus strains is added to the feed in an amount required to administer at least 10⁴-10⁸ CFU, preferably 10⁵-10⁸ CFU, more preferably 10⁵-10⁷ CFU per kg body weight of an individual animal per day.

More specifically, in the manufacture of an animal feed in accordance with the invention, from about 10⁷ CFU /kg of one or more probiotic Bacillus strains, preferably from about 10⁷ CFU /kg to about 10⁹ CFU /kg of one or more probiotic Bacillus strains, most preferably from about 10⁷ CFU/kg to about 10⁹ CFU/kg of one or more probiotic Bacillus strains are suitably added to regular animal feed.

In a further embodiment, the invention is a feed wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed, dependent on factors such as species and age.

More specifically, the invention is a feed wherein the vitamin E content is reduced below 300 mg/kg feed, preferably below 250 mg/kg feed, preferably below 200 mg/kg feed, more preferably below 150 mg/kg feed and most preferably below 100 mg/kg feed for broiler starters.

More specifically, the invention is a feed wherein the vitamin E content is reduced below 100 mg/kg feed, more preferably below 50 mg/kg feed for broiler growers and broiler finishers.

More specifically, the invention is a feed wherein the vitamin E content is reduced below 100 mg/kg feed, more preferably below 80 mg/kg feed for broiler growers and broiler breeder starters and growers.

More specifically, the invention is a feed wherein the vitamin E content is reduced below 150 mg/kg feed, more preferably below 100 mg/kg feed for broiler growers and broiler breeder layers and male breeders.

More specifically, the invention is a feed wherein the vitamin E content is reduced below 100 mg/kg feed, preferably below 50 mg/kg feed and most preferably below 40 mg/kg feed for Hen and duck starters.

More specifically, the invention is a feed wherein the vitamin E content is reduced below 35 mg/kg feed, preferably below 30 mg/kg feed for Hen and duck rearers.

More specifically, the invention is a feed wherein the vitamin E content is reduced below 30 mg/kg feed, preferably below 25 mg/kg feed and most preferably below 20 mg/kg feed for Hen and duck layers.

More specifically, the invention is a feed wherein the vitamin E content is reduced below 100 mg/kg feed, more preferably below 50 mg/kg feed for layer breeders (growers, layers and male breeders).

More specifically, the invention is a feed wherein the vitamin E content is reduced below 80 mg/kg feed, preferably below 50 mg/kg feed and most preferably below 40 mg/kg feed for duck and geese.

More specifically, the invention is a feed wherein the vitamin E content is reduced below 80 mg/kg feed, preferably below 50 mg/kg feed for Partridges, quails and pheasants.

More specifically, the invention is a feed wherein the vitamin E content is reduced below 60 mg/kg feed, preferably below 50 mg/kg feed and most preferably below 40 mg/kg feed for ostriches and emus.

More specifically, the invention is a feed wherein the vitamin E content is reduced below 250 mg/kg feed, preferably below 200 mg/kg feed, preferably below 150 mg/kg feed, and most preferably below 100 mg/kg feed for turkey starters.

More specifically, the invention is a feed wherein the vitamin E content is reduced below 80 mg/kg feed, preferably below 60 mg/kg feed for turkey growers.

More specifically, the invention is a feed wherein the vitamin E content is reduced below 50 mg/kg feed, preferably below 30 mg/kg feed for turkey finishers.

More specifically, the invention is a feed wherein the vitamin E content is reduced below 250 mg/kg feed, preferably below 200 mg/kg feed, preferably below 150 mg/kg feed, and most preferably below 100 mg/kg feed for turkey breeder starters.

More specifically, the invention is a feed wherein the vitamin E content is reduced below 80 mg/kg feed, preferably below 60 mg/kg feed for turkey breeder growers.

More specifically, the invention is a feed wherein the vitamin E content is reduced below 150 mg/kg feed, preferably below 100 mg/kg feed for turkey breeder layers and male breeders.

More specifically, the invention is a feed wherein the vitamin E content is reduced below 150 mg/kg feed, preferably below 100 mg/kg feed for fattening pig pre-starters and starters.

More specifically, the invention is a feed wherein the vitamin E content is reduced below 100 mg/kg feed, preferably below 60 mg/kg feed for fattening pig growers and finishers.

More specifically, the invention is a feed wherein the vitamin E content is reduced below 100 mg/kg feed, preferably below 80 mg/kg feed for swine breeder replacement gilts.

More specifically, the invention is a feed wherein the vitamin E content is reduced below 150 mg/kg feed, preferably below 100 mg/kg feed for sows and boars.

In a further preferred embodiment of the present invention the composition is a feed premix, i.e. one or more probiotic Bacillus strains according to the present invention are - e.g. as a formulated powder - added to other minerals, vitamins, amino acids and/or trace elements in a higher concentration in order to form the premix. For use the premix is added to and thoroughly mixed with a regular animal feed to achieve even distribution therein. Such a premix can comprise active ingredients, e.g., in a concentration of from about 1 mg/kg to about 10 000 mg/kg, preferably from about 100 mg/kg to about 10 000 mg/kg, more preferably from about 200 mg/kg to about 8 000 mg/kg, most preferably from about 500 mg/kg to about 7 000 mg/kg of one or more probiotic Bacillus strains. If one kg of such premix is added per 100 kg of regular feed this would typically meet the individual need of the animal by normal feed consumption.

For optimum results, the probiotic Bacillus strain supplementation should be part of a diet which contains an adequate supply of nutrients, generally known to be of benefit to the organism.

If more than one probiotic Bacillus strain is added to the feed composition, their individual amounts in a dry food composition should be adapted accordingly.

According to the present invention it is further advantageous if the composition also contains one or more of the following ingredients: Vitamin A, Vitamin E, Biotin, copper (e.g. as CuSO₄), zinc (e.g. as ZnSO₄), cobalt (e.g. as CoSO₄), selenium (e.g. as Na₂SeO₃), iodine (e.g. as KI), manganese (e.g. as MnSO₄) and/or calcium (e.g. as CaSO₄). It is preferred to use calcium sulphate as calcium carrier in a composition according to the present invention.

When the composition is prepared in the form of a premix the premix preferably comprises from 10⁹ CFU/g composition, preferably in an amount from 10⁸ CFU/g composition, most preferably in an amount from 2x10⁸ CFU/g composition one or more probiotic Bacillus strains, and further from 80,000 to 120,000 IU Vitamin A, from 1000 to 3000 IU Vitamin E, from 10 to 20 mg Biotin, from 200 to 300 mg copper (as CuSO₄), from 300 to 600 mg zinc (as ZnSO₄), from 5 to 10 mg cobalt (as CoSO₄), from 1 to 6 mg selenium (as Na₂SeO₃), from 5 to 10 mg iodine (as KI) and/or from 200 to 400 mg manganese (as MnSO₄) and may further comprise magnesium chloride or sulphate and sodium bicarbonate.

According to the present invention it is preferred to start administering one or more probiotic Bacillus strains at weaning.

### DEFINITIONS

**BioPlus® YC:** A mixture of *Bacillus licheniformis* DSMZ 5749 und *Bacillus subtilis* DSMZ 5750 is available as a commercial formulation under the Trademark BioPlus® YC.

**Feed Additive:** The term feed additive according to the invention refers to a formulation comprising one or more probiotic Bacillus strains chosen from the group of Bacillus subtilis and Bacillus licheniformis as active ingredient intended for intake by the animal.

**Feed Premix:** The incorporation of the composition of feed additives to animal feeds, is in practice carried out using a concentrate or a premix. A premix designates a preferably uniform mixture of one or more microingredients with diluent and/or carrier. Premixes are used to facilitate uniform dispersion of micro-ingredients in a larger mix. A premix according to the invention can be added to feed ingredients as solids (for example as water soluble powder) or liquids.

**Feed:** The term "Feed" refers to any compound, preparation, or mixture suitable for, or intended for intake by animals. The terms "feed" or "feed composition" as used herein comprise solid and liquid food as well as drinking fluids such as drinking water.

An animal feed for pigs typically comprises proteins, carbohydrates, fats, further probiotics, prebiotics, enzymes, vitamins, immune modulators, milk replacers, minerals, amino acids, acid-based products, medicines, and/or other combinations thereof. Typical feed ingredients include corn, Soybean meal, Soy protein, Potato proteins, Lactoserum, Soybean oil, fish meal, krill meal, soya concentrate, corn gluten, wheat gluten, pea protein, wheat flour, fish oil, a vitamin, mineral premix, mineral premix plus synthetic phosphorus and combinations thereof.

The feed refers to a manufactured or artificial diet (i.e., formulated feed) to supplement or to replace natural feed, which is most commonly produced in form of mash or pellets. Such a feed typically comprises a carbohydrate source, a protein source and/or a lipid source, in the amounts of 15-30 % (w/w) of a protein source, 65-80 % (w/w) of a carbohydrate source and/or 3-5 % (w/w) of a lipid source. The feed may further comprise ash, water and/or any combinations thereof. The feed may further comprise one or more ingredients selected from the group consisting of, a vitamin, mineral premix and combinations thereof.

**Mutant strain:** The term "mutant strain" used throughout refers to mutants that arise from a wild type *Bacillus,* such as BMS5 whose genome is given in SEQ ID NO:1, with the intentional use of mutagens such as those known in the art, and preferably EMS. Such mutants may be obtained by classical methods, such as growing the *Bacillus subtilis* strain in the presence of UV light or in the presence of a certain antibiotic to which the parent is susceptible and testing any resistant mutants for improved biological activity or improved ability to enhance one or more of the indicia of animal health. Other methods for identifying mutants are known to those of ordinary skill in the art. But besides these preferred mutants all other kinds of mutants e.g., mutants obtained by genetic engineering, are also part of the current invention. The mutant strains of the current invention are preferably mutants of existing strains.

**BMS1:** BMS1 is a mutant strain from *Bacillus licheniformis* DSMZ 5749 possessing mutations alsS and dhbA (as defined below).
alsS (*B*. *licheniformis* BMS1) (wild type) 
lnnvaaknetltvrgaelvvdsliqqgvthvfgipgakidavfdvlkdkgpelivcrheqnaafmaaavgrltgkpgvclvtsgpgas
nlatglvtantegdpvvalagavkradrlkkthqsmdnaalfqPITKYSAEVEDANNIPEAVTNAFRAAASGQAGAAF
LSFPQDVTAGPATAKPVKTMPAPKLGAASDEQISAAIAKIHNANLPVVLVGMKGGRPEAIEAVRRLLRKV
KLPFVETYQAAGTLSHDLEDQYFGRIGLFRNQPGDMLLEKADVVLTVGYDPIEYDPVFWNGKGERSVIHL
DEIQADIDHDYQPEIELIGDIAETLNHIEHDSLPVSIDESFAPVLDYLKKALEEQSEPPKETKTDLVHPLQIVR
DLRELLSDDITVTCDIGSHAIWMSRYFRTYRPHGLLISNGMQTLGVALPWAIAATLVNPGQKVVSVSGDG
GFLFSAMELETAVRLKAPIVHIVWNDSTYDMVAFQQEMKYKRTSGVDFGGIDIVKYAESFGAKGLRVNS
PDELAEVLKAGLDAEGPVVIDIPVDYSDNIHLADQRFPKKFEEHFNKEASKQS*
dhbA (*B. licheniformis* BMS1) (wild type)

vkgkvalvtgasqgigkevalalagrgvfvaaadqnrkgllelede leqkglqgsgfaadvgdsaavdqliadiereigpidmlvnv

agvlrtglihslsdedwektfnvnstgvfnvsravarrmvprrtgaivtvgsnaaavprmhmaayaaskaaalmftkclglelaey

nircniispgstdtpmqrslwqceeaaqgviegsletfktgiplgklaspadiadavvfllsdgarhitmhd

**BMS2:** BMS2 is a *Bacillus licheniformis* strain with genome deposited under NRRL B-50134.

**BMS3:** BMS3 is a *Bacillus subtilis* strain with genome deposited under NRRL B-50105.

**BMS4:** BMS4 is a *Bacillus subtilis* strain with genome published under code NC_019896 (Schyns et al., 2013 in Genome announcements).

**BMS5:** BMS5 is a wild type *Bacillus subtilis* whose genome is **SEQ.ID.NO.:1.** Due to its length, it is not reproduced in print.

### Brief description of the figures

Figure 1. Vitamin E absorption in plasma comparing weaned piglets fed control diet or a feed comprising dietary probiotic Bacillus strains at 5x10⁸ CFU/kg of feed compared to control basal diet fed pigs.
Figure 2. Vitamin E absorption in plasma comparing weaned piglets fed control diet or a feed comprising dietary probiotic Bacillus strains at different concentrations compared to control basal diet fed pigs.

### Detailed description of the invention

1. The use of at least one Bacillus strain for improving vitamin E absorption in animals.
2. The use of claim 1 wherein the animal is a monogastric animal.
3. The use of claim 2 wherein the animal is a swine.
4. The use of claim 3 wherein the animal is a weaned piglet.
5. The Bacillus strain according to any of claims 1 to 4 wherein the strain is selected from the group of Bacillus subtilis and Bacillus licheniformis.
6. The Bacillus strain according to any of claims 1 to 4 wherein the Bacillus strain composition comprises a strain chosen from the group of Bacillus subtilis BMS 3, BMS 4, BMS 5, DSMZ 5750 or Bacillus licheniformis BMS 1, BMS 2, DSMZ 5749 or a mutant of any of these strains.
7. The use according to any of claims 1 to 6, wherein said Bacillus strain is administered in an amount of at least 10⁷ CFU/kg feed, preferably 10⁷-10¹⁰ CFU/kg feed, more preferably 10⁷-10⁹ CFU/kg feed.
8. The use according to any of claims 1 to 7, wherein said Bacillus strain is administered in an amount of at least 10⁴-10⁸ CFU/kg body weight per day, preferably 10⁵-10⁸ CFU/kg body weight per day, more preferably 10⁵-10⁷ CFU/kg body weight per day.
9. A method for improving vitamin E absorption in animals, said method comprises feeding to the animal at least one Bacillus strain.
10. The method of claim 9 wherein the animal is a monogastric animal.
11. The method of claim 10 wherein the animal is a swine.
12. The method of claim 11 wherein the animal is a weaned piglet.
13. The method according to any of claims 9 to 12 wherein the strain is selected from the group of Bacillus subtilis and Bacillus licheniformis.
14. The method according to any of claims 9 to 13 wherein the Bacillus strain composition comprises a strain chosen from the group of Bacillus subtilis BMS 3, BMS 4, BMS 5, DSMZ 5750 or Bacillus licheniformis BMS 1, BMS 2, DSMZ 5749 or a mutant of any of these strains.
15. The method according to any of claims 9 to 14, wherein said Bacillus strain is administered in an amount of at least 10⁷ CFU/kg feed, preferably 10⁷-10¹⁰ CFU/kg feed, more preferably 10⁷-10⁹ CFU/kg feed.
16. The method according to any of claims 9 to 15, wherein said Bacillus strain is administered in an amount of at least 10⁴-10⁸ CFU/kg body weight per day, preferably 10⁵-10⁸ CFU/kg body weight per day, more preferably 10⁵-10⁷ CFU/kg body weight per day.
17. An animal feed composition comprising at least one Bacillus strain of claim 1 or compounds obtained from the Bacillus of claim 1 for improving vitamin E absorption in animals.
18. The feed composition of claim 17 wherein the animal is a monogastric animal.
19. The feed composition of claim 18 wherein the animal is a swine.
20. The feed composition of claim 19 wherein the animal is a weaned piglet.
21. The feed composition according to any of claims 17 to 20 comprising the Bacillus strain of claim 1 or compounds obtained from the Bacillus of claim 1 in a concentration of at least 10⁷ CFU/kg feed, preferably 10⁷-10¹⁰ CFU/kg feed, more preferably 10⁷-10⁹ CFU/kg feed.
22. The feed composition according to any of claims 17 to 21, wherein the feed composition further comprises one or more ingredients from the group consisting of a carbohydrate source, a protein source, a lipid source, ash, water and any combinations thereof and wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed.
23. The feed composition according to any of claims 17 to 21, comprising at least one further ingredient selected from the group consisting of: proteins, carbohydrates, fats, further probiotics, prebiotics, enzymes, vitamins, immune modulators, milk replacers, minerals, amino acids, acid-based products, medicines, and combinations thereof and wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed.
24. The feed composition according to any of claims 17 to 21, comprising at least one further ingredient selected from the group consisting of: corn, Soybean meal, Soy protein, Potato proteins, Lactoserum, Soybean oil, fish meal, krill meal, soya concentrate, corn gluten, wheat gluten, pea protein, wheat flour, fish oil, a vitamin, mineral premix, mineral premix plus synthetic phosphorus and combinations thereof and wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed.
25. The feed composition according to any claims 17 to 24, wherein the feed composition comprises from 15-30 % (w/w) of a protein source.
26. The feed composition according to claim 17 to 24, wherein the feed composition comprises from 65-80 % (w/w) of a carbohydrate source.
27. The feed composition according to claim 17 to 24, wherein the feed composition comprises from 3-5 % (w/w) of a lipid source.

The invention is further illustrated by the following examples.

### Example 1

The first study evaluated dietary probiotic Bacillus strains at 5x10⁸ CFU/kg of feed compared to control basal diet fed pigs.
144 male piglets (Large-White x Redon) were divided in groups of 4 piglets per treatment with 6 replicates per treatment (24 piglets/treatment) and fed pellets of sample or control diets ad libitum for 42 days. Vitamin E absorption in the plasma was measured after 21 days of treatment.

Treatment dosages are shown in table 1 and diet composition in table 2.

**Table 1 Dosage [CFU/kg feed] of probiotic in treatments**

| Treatment | Control | BMS1 | BMS2 | BMS3 | BMS4 | BMS5 |
|---|---|---|---|---|---|---|
| Dose [CFU/kg feed] | 0 | 5x10⁸ | 5x10⁸ | 5x10⁸ | 5x10⁸ | 5x10⁸ |

**Table 2 Composition of the diets**

| Phase | D1-7 | D7-21 | D21-42 |
|---|---|---|---|
| **Ingredient inclusion (%)** | | | |
| Corn | 52.80 | 55.05 | 60.20 |
| Soybean meal CP48 | 26.50 | 26.50 | 29.50 |
| Soy protein concentrate | 3.00 | 3.00 | 1.00 |
| Potato proteins | 2.00 | 2.00 | 0 |
| Lactoserum | 5.00 | 3.00 | 0 |
| Soybean oil | 4.15 | 4.15 | 3.00 |
| Vitamin &Trace Mineral Premix | 3.00 | 3.00 | 3.00 |
| Dicalcium phosphate | 1.90 | 1.90 | 1.90 |
| Calcium carbonate | 0.60 | 0.60 | 0.60 |
| L-Lys | 0.70 | 0.50 | 0.50 |
| L-Met | 0.15 | 0.10 | 0.10 |
| L-Thr | 0.05 | 0.05 | 0.05 |
| L-Trp | 0.05 | 0.05 | 0.05 |
| NaCl | 0.10 | 0.10 | 0.10 |
| Phytase (mg/kg) | 100 | 100 | 100 |

| **Calculated Provision** | | | |
|---|---|---|---|
| CP (%) | 20.6 | 20.4 | 19.1 |
| Metabolizable Energy (MJ/kg) | 13.06 | 13.04 | 12.72 |

| **Analyzed content** | | | |
|---|---|---|---|
| CP (%) | 21.27 ± 0.13 | 21.35 ± 0.24 | 19.25 ± 0.51 |
| Metabolizable Energy (MJ/kg) | 14.79 ± 0.16 | 14.80 ± 0.13 | 14.37 ± 0.34 |

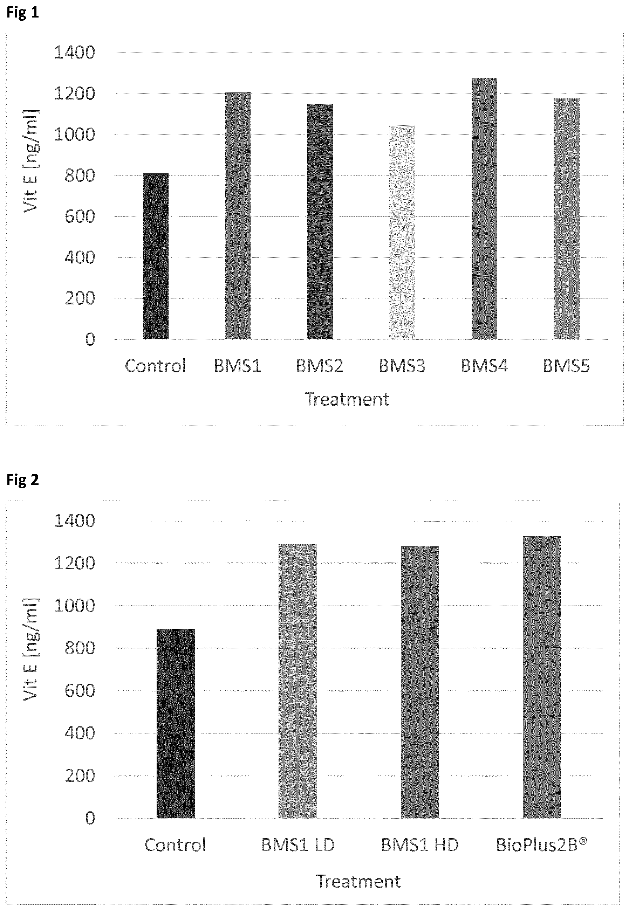

The results are shown in Table 3 and Figure 1. The probiotic treatment resulted in an increased vitamin E concentration in the plasma of at least 63%.

**Table 3 Vitamin E content in plasma**

| | Vit E in plasma [ng/ml] | Increase in vitamine E [%] |
|---|---|---|
| Control | 810 | 0 |
| BMS1 | 1208 | 67 |
| BMS2 | 1152 | 70 |
| BMS3 | 1051 | 77 |
| BMS4 | 1276 | 63 |
| BMS5 | 1177 | 69 |

### Example 2

The second study evaluated dietary probiotic Bacillus strains at different dosages compared to control basal diet fed pigs.
144 male piglets (Large-White x Redon) were divided in groups of 4 piglets per treatment with 9 replicates per treatment (36 piglets/treatment) and fed pellets of sample or control diets ad libitum for 42 days. Vitamin E absorption in the plasma was measured after 21 days of treatment.

Treatment dosages are shown in table 3 and diet composition in table 4.

**Table 4 Dosage [CFU/kg feed] of probiotic in treatments**

| Treatment | Control | BMS1 HD | BMS1 LD | BioPlus2B® |
|---|---|---|---|---|
| Dose [CFU/kg feed] | 0 | 5x10⁸ | 1.6x10⁸ | 1.6x10⁸ |

**Table 5 Composition of the diets**

| Phase | D1-7 | D7-21 | D21-42 |
|---|---|---|---|
| **Ingredient inclusion (%)** | | | |
| Corn | 50.80 | 54.65 | 61.60 |
| Soybean meal CP48 | 28.00 | 28.40 | 29.00 |
| Soy protein concentrate | 5.00 | 3.00 | 2.00 |
| Potato proteins | 2.00 | 2.00 | 0 |
| Lactoserum | 5.00 | 3.00 | 0 |
| Soybean oil | 3.00 | 3.00 | 1.50 |
| Vitamin &Trace Mineral Premix | 3.00 | 3.00 | 3.00 |
| Dicalcium phosphate | 1.70 | 1.55 | 1.40 |
| Calcium carbonate | 0.60 | 0.60 | 0.70 |
| L-Lys | 0.60 | 0.50 | 0.50 |
| L-Met | 0.10 | 0.10 | 0.10 |
| L-Thr | 0.05 | 0.05 | 0.05 |
| L-Trp | 0.05 | 0.05 | 0.05 |
| NaCl | 0.10 | 0.10 | 0.10 |
| Phytase (mg/kg) | 100 | 100 | 100 |
| Chromium oxide | No | No | Yes |

| **Calculated Provision** | | | |
|---|---|---|---|
| CP (%) | 21.7 | 20.6 | 18.9 |
| Metabolizable Energy (MJ/kg) | 13.45 | 13.52 | 13.30 |

| **Analyzed content** | | | |
|---|---|---|---|
| CP (%) | 22.7 ± 0.2 | 21.4 ± 0.4 | 19.9 ± 0.4 |
| Metabolizable Energy (MJ/kg) | 13.33 ± 0.1 | 13.311276 ± 0.1 | 13,09 ± 0.1 |

The results are shown in Table 6 and Figure 2. The probiotic treatment resulted in an increased vitamin E concentration in the plasma of at least 67%.

**Table 6 Vitamin E content in plasma**

| | Vit E in plasma [ng/ml] | Increase in vitamine E [%] |
|---|---|---|
| Control | 893 | 0 |
| BMS1 LD | 1289 | 69 |
| BMS1HD | 1279 | 70 |
| BioPlus2B® | 1328 | 67 |

### Example 3

Recommended vitamin E contents for poultry are shown in table 7.

**Table 7 Recommended vitamin E content in poultry feed**

| Category | Phase | Duration | Vit. E [mg/kg feed] ⁽¹⁾ |
|---|---|---|---|
| Broilers | Starters | 1 - 10 days | 150 - 300 ⁽²⁾ |
| | Growers | 11- 24 days | 50- 100 ⁽³⁾ |
| | Finishers | 25 days - market | 50 - 100 ⁽³⁾⁽⁴⁾ |
| Broiler breeders | Starters & growers | 0-18 wks | 80 - 100 ⁽²⁾ |
| | Layers & male breeders | 19 wks - end | 100 - 150 ⁽³⁾ |
| Hen & duck layers | Starters (pullets) | 0-10 wks | 40 - 100 ⁽²⁾ |
| | Rearing (pullets) | 10 wks - 2% lay | 30 - 35 |
| | Layers | Laying phase | 20 - 30 ⁽³⁾ |
| Layer breeders | Growers, layers & male breeders | 0 wks - end | 50 - 100 ⁽³⁾ |
| Ducks & geese | | | 40 - 80 |
| Partridged, quails & pheasants | | | 50 - 80 |
| Ostriches & emus | | | 40 - 60 |
| Turkeys | Starters | 0 - 6 wks | 100 - 250 ⁽²⁾ |
| | Growers | 7 - 12 wks | 60 - 80 |
| | Finishers 1 | 13 - 18 wks | 30 - 50 ⁽³⁾⁽⁴⁾ |
| | Finishers 2 | 18 wks - market | 30 - 50 ⁽³⁾⁽⁴⁾ |
| Turkey breeders | Starters | 0 - 6 wks | 100 - 250 ⁽²⁾ |
| | Growers | 7 - 29 wks | 60 - 80 |
| | Layers & male breeders | Laying phase | 100 - 150 ⁽³⁾ |

| | | | |
|---|---|---|---|
| ⁽¹⁾ When dietary fat is higher than 3% then add 5 mg/kg feed for each 1% dietary fat. ⁽²⁾ Higher level for optimum function. ⁽³⁾ Under heat stress conditions increase level up to 200 mg/kg feed. ⁽⁴⁾ For optimum meat quality increase level up to 200 mg/kg feed. | | | |

### Example 4

Recommended vitamin E contents for swine are shown in table 8.

**Table 8 Recommended vitamin E content in swine feed**

| Category | Phase | Duration | Vit. E [mg/kg feed] ⁽¹⁾ |
|---|---|---|---|
| Fattening pigs | Pre-starters | < 5 kg | 100 - 150 ⁽²⁾ |
| | Starters | 5 - 30 kg | 100 - 150 |
| | Growers | 30 - 70 kg | 60 - 100 |
| | Finishers | 70 kg - market | 60 - 100 ⁽³⁾ |
| Breeders | Replacement gilts | | 80 - 100 |
| | Sows | | 100 - 150 ⁽⁴⁾ |
| | Boars | | 100 - 150 |

| | | | |
|---|---|---|---|
| ⁽¹⁾ When dietary fat is higher than 3% then add 5 mg/kg feed for each 1% dietary fat. ⁽²⁾ For optimum immune health: additional 100 mg/kg feed. ⁽³⁾ For optimum meat quality: additional 150 mg/kg feed. ⁽⁴⁾ For optimum piglet health: during late pregnancy and lactation total 250 mg/kg feed. | | | |

## Claims

1. The use of at least one Bacillus strain for improving vitamin E absorption in animals.

2. The use of claim 1 wherein the animal is a monogastric animal.

3. The use of claim 2 wherein the animal is a swine.

4. The use of claim 3 wherein the animal is a weaned piglet.

5. The Bacillus strain according to any of claims 1 to 4 wherein the strain is selected from the group of Bacillus subtilis and Bacillus licheniformis.

6. The Bacillus strain according to any of claims 1 to 4 wherein the Bacillus strain composition comprises a strain chosen from the group of Bacillus subtilis BMS 3, BMS 4, BMS 5, DSMZ 5750 or Bacillus licheniformis BMS 1, BMS 2, DSMZ 5749 or a mutant of any of these strains.

7. The use according to any of claims 1 to 6, wherein said Bacillus strain is administered in an amount of at least 10⁷ CFU/kg feed, preferably 10⁷-10¹⁰ CFU/kg feed, more preferably 10⁷-10⁹ CFU/kg feed.

8. The use according to any of claims 1 to 7, wherein said Bacillus strain is administered in an amount of at least 10⁴-10⁸ CFU/kg body weight per day, preferably 10⁵-10⁸ CFU/kg body weight per day, more preferably 10⁵-10⁷ CFU/kg body weight per day.

9. A method for improving vitamin E absorption in animals, said method comprises feeding to the animal at least one Bacillus strain.

10. The method of claim 9 wherein the animal is a monogastric animal.

11. The method of claim 10 wherein the animal is a swine.

12. The method of claim 11 wherein the animal is a weaned piglet.

13. The method according to any of claims 9 to 12 wherein the strain is selected from the group of Bacillus subtilis and Bacillus licheniformis.

14. The method according to any of claims 9 to 13 wherein the Bacillus strain composition comprises a strain chosen from the group of Bacillus subtilis BMS 3, BMS 4, BMS 5, DSMZ 5750 or Bacillus licheniformis BMS 1, BMS 2, DSMZ 5749 or a mutant of any of these strains.

15. The method according to any of claims 9 to 14, wherein said Bacillus strain is administered in an amount of at least 10⁷ CFU/kg feed, preferably 10⁷-10¹⁰ CFU/kg feed, more preferably 10⁷-10⁹ CFU/kg feed.

16. The method according to any of claims 9 to 15, wherein said Bacillus strain is administered in an amount of at least 10⁴-10⁸ CFU/kg body weight per day, preferably 10⁵-10⁸ CFU/kg body weight per day, more preferably 10⁵-10⁷ CFU/kg body weight per day.

17. An animal feed composition comprising at least one Bacillus strain of claim 1 or compounds obtained from the Bacillus of claim 1 for improving vitamin E absorption in animals.

18. The feed composition of claim 17 wherein the animal is a monogastric animal.

19. The feed composition of claim 18 wherein the animal is a swine.

20. The feed composition of claim 19 wherein the animal is a weaned piglet.

21. The feed composition according to any of claims 17 to 20 comprising the Bacillus strain of claim 1 or compounds obtained from the Bacillus of claim 1 in a concentration of at least 10⁷ CFU/kg feed, preferably 10⁷-10¹⁰ CFU/kg feed, more preferably 10⁷-10⁹ CFU/kg feed.

22. The feed composition according to any of claims 17 to 21, wherein the feed composition further comprises one or more ingredients from the group consisting of a carbohydrate source, a protein source, a lipid source, ash, water and any combinations thereof and wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed.

23. The feed composition according to any of claims 17 to 21, comprising at least one further ingredient selected from the group consisting of: proteins, carbohydrates, fats, further probiotics, prebiotics, enzymes, vitamins, immune modulators, milk replacers, minerals, amino acids, acid-based products, medicines, and combinations thereof and wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed.

24. The feed composition according to any of claims 17 to 21, comprising at least one further ingredient selected from the group consisting of: corn, Soybean meal, Soy protein, Potato proteins, Lactoserum, Soybean oil, fish meal, krill meal, soya concentrate, corn gluten, wheat gluten, pea protein, wheat flour, fish oil, a vitamin, mineral premix, mineral premix plus synthetic phosphorus and combinations thereof and wherein the vitamin E content is reduced below 95%, preferably below 90%, preferably below 85%, preferably below 80 %, more preferably below 75%, more preferably below 70%, more preferably below 65%%, more preferably below 60%, more preferably below 55%, more preferably below 50%, more preferably below 45%, more preferably below 40%, more preferably below 35%, more preferably below 30% of the recommended standard values in animal feed.

25. The feed composition according to any claims 17 to 24, wherein the feed composition comprises from 15-30 % (w/w) of a protein source.

26. The feed composition according to claim 17 to 24, wherein the feed composition comprises from 65-80 % (w/w) of a carbohydrate source.

27. The feed composition according to claim 17 to 24, wherein the feed composition comprises from 3-5 % (w/w) of a lipid source.
